# EUROPEAN PATENT APPLICATION

(11) **EP 0 761 232 A1**
(43) Date of publication of application: **12.03.1997**
(21) Application number: 96306510.7
(22) Date of filing: 06.09.1996
(51) Int. Cl.: A61K 39/395

(54) **Antiallergic agent comprising anti-PAF receptor antibodies**

(30) Priority: 08.09.1995 JP 257023/95
(71) Applicant: Sumitomo Electric Industries, Ltd., Osaka 541 (JP)
(72) Inventor: Nakata, Motomi, Sumitomo Elect. Ind., Ltd., Yokohama-shi, kanagawa (JP); Sagara, Hironori, Mibu, Shimotsuga-gun, Tochigi (JP); Makino, Sho-hei, Mibu, Shinotsuga-gun, Tochigi (JP); Okumura, Ko, Chuo-ku, Chiba-shi, Chiba (JP)
(74) Representative: Perry, Robert Edward

(57) **Abstract**

An antibody against a human PAF receptor or an active fragment thereof, is an anti-allergic agent. It is particularly useful for the treatment for allergic bronchial asthma.

## Description

### FIELD OF THE INVENTION

The present invention relates to an antiallergic agent, and more particularly to an antiallergic agent comprising, as an active ingredient, an antibody against a human PAF receptor or an active fragment thereof. The antiallergic agent according to the present invention is particularly useful for treatment for allergic bronchial asthma among allergic diseases.

### BACKGROUND OF THE INVENTION

Bronchial asthma has heretofore been understood being a disease caused by the type I allergic reaction among 4 types of allergic reactions classified by Gell and Coombs. More specifically, IgE coupled to an antigen couples to FCεRI situated on a surface of a mast cell, whereby the mast cell is activated, so that inflammatory mediators such as histamine and leukotriene are released. It has been said that by these mediators, vascular permeability acceleration, smooth muscle constriction and gland-secretion acceleration are brought on, resulting in obstruction of airway. However, only the immediate allergic reaction (immediate asthmatic response) is caused by this reaction. Therefore, a delayed allergic reaction (late asthmatic response), which takes place after 6 hours to 10 hours, has been unable to be explained.

In recent years, various facts regarding this have been understood from autopsic findings of death from asthma. Namely, obstruction of a respiratory lumen with mucous plug, ablation of bronchial epithelium and cellular infiltration of, mainly, eosinophils and monocytes have been recognized. The same has been said of a mild case. Bronchial asthma has recently come to be regarded as an chronic inflammation caused by CD4⁺-T cells and eosinophils by the observation through a bronchoscope or the like. At present, according to the guide line on treatment for asthma of the Japanese Association of Allergology, it is said that "bronchial asthma is characterized by wide range and various degrees of airway obstruction and airway inflammation. The airway inflammation is involved by many inflammatory cells such as lymphocytes, eosinophils and mast cells, shows damage of a respiratory mucous epithelium and is accompanied by the acceleration of airway reactivity to various stimulations.".

In recent years, it has been considered that how a late asthmatic response is inhibited is an important problem in bronchial asthma, and its analysis has come to be advanced. In this regard, its details are described in "Bronchial Asthma Viewed from Molecular Biology" published by K.K. Gendai Iryo-sha. More specifically, the said book explains that "the fact that inflammation plays an important part in the formation of morbid state of allergic diseases including bronchial asthma has come to be recognized. Inflammatory cells include cells such as mast cells, eosinophils, neutrophils, lymphocytes and macrophages. In allergic inflammation, attention is paid to eosinophils among these cells. Eosinophils are considered to be the center of late asthmatic response, and locally accumulated at inflammatory parts of bronchial asthma by the cooperative action of an eosinophil chemotactic factor (ECF) and cytokine. Eosinophils mobilized to bronchial mucosa are known to cause a disorder of bronchial epithelium and accelerate airway reactivity. This is considered to be attributable to the fact that MBP and ECP which are specific granules for eosinophils have a tissue-damaging action and further facilitate histamine release from mast cells and basophils, or eosinophils themselves produce strong inflammatory mediators such as activating enzymes, LTC₄ and PAF to participate in airway contraction and mucosal ablation. For this reason, eosinophils have recently been considered to play a central part in to make bronchial asthma chronic and prolonged. More recently, the antigen-presenting ability of eosinophils have been proved, and attention has been paid to a role as immunocompetent cells." (page 328).

These analyses suggest that if eosinophils can be successfully controlled, there is a possibility that bronchial asthma may be inhibited. Namely, the said book explains that "In patients of bronchial asthma, expectoration, increase of eosinophils in peripheral blood, and conglomeration of eosinophils into the lungs even in examples of autopsy have been well known before now. At present, eosinophils are recognized as an effector for LAR (late asthmatic response).

According to a biopsy on the bronchial mucosa of a bronchial asthma patient, it is indicated that activated eosinophils (EG²⁺) and major basic protein (MBP) exist, and the number of eosinophils and the condition of a patient correlate to each other.

Eosinophils degranulate in secretory IgA, IgG, PAF, C3b, C3bi, IL-5, GM-CSF, substance P, FMLP and the like, namely, release MBP, eosinophil cationic protein (ECP), eosinophil derived neurotoxin (EDN) and eosinophil peroxidase (EOP). MBP causes epithelial ablation when administrated to human respiratory epithelium. They also release LTC₄, PAF, peroxides. These have activities to airway tissue damage, and besides, smooth muscle contraction, vascular permeability acceleration, muciparous acceleration and inflammatory cell migration. As described above, tissue damage is brought on by eosinophils.

With respect to airway hypersensitivity, it is recognized that the numbers of eosinophils, MBP and ablated respiratory epithelia in a broncoalveolar lavage fluid (BALF) and PC20 correlate to each other. With respect to the mechanism of airway hypersensitivity acceleration, ablation of respiratory epithelium, exposure of sensory nerve terminal by lesion, epithelial permeability acceleration and the like are considered. As described above, as with the airway tissue damage, eosinophils are considered to be effector cells even in the airway hypersensitivity acceleration.

Recently, it has been known that eosinophils can fill another role than the fact that they directly cause inflammation in allergic inflammation. Eosinophils are activated by IL-3, IL-5 and GM-CSF, and also produce these cytokines by themselves. MBP and MPO not only cause tissue damage, but also bring on histamine release from mast cells. They have features as APC (antigen presenting cell), namely, react to IL-4, IFN-γ and GM-CS to express HLA-DR and ICAM-1 and to produce IL-1α. It has not been yet known that these actually contribute to bronchial asthma to what degree." (page 40).

### OBJECTS AND SUMMARY OF THE INVENTION

The present inventors have paid attention to a platelet-activating factor (PAF) as a substance which bears migration and activation of these eosinophils. PAF is a phospholipid found as a factor derived from a leukocyte and inducing the activation of platelets, for example, shape change, histamine release and aggregation, and has a structure of 1-O-alkyl-2-acetyl-sn-glycero-3-phosphocholine.

PAF is a phospholipid autacoid produced in platelets, leukocytes, lung, spleen, kidney, brain, etc., has potent inflaming effect and hypotensive effect in a small amount, and is considered to participate in bronchial asthma, endotoxin shock, anaphylactic shock and the like. More specifically, PAF has not only a platelet-activating effect, but also neutrophil-activating effect, macrophage-activating effect, hypotensive effect, vascular permeability-accelerating effect, smooth muscle-contracting effect, glycogenolysis-accelerating effect in the liver, and the like, and is a factor involved in the development of anaphylaxis, inflammation, allergy and the like in vivo. It has been found that PAF activates a target cell such as a platelet, neutrophil or macrophage through a receptor on a cell membrane and also has an effect to migrate or activate eosinophils.

The presence of this physiologically active phospholipid was confirmed in 1972, and its structure was clarified in 1979. However, the analysis of the PAF receptor has not been very developed up to date. The reasons for it are considered, for example, to be as follows. Since PAF is lipophilic and high in nonspecific affinity for cell membranes, even a basic ligand binding experiment is difficult to conduct. Besides, since a receptor protein exists only in a small amount in a cell membrane, its purification is difficult.

Shimizu *et al*, Nature 349:342 (1991), report isolating cDNAs of the PAF receptor from the guinea pig lung and human leukocytes using a molecular biology technique. From the structure of the PAF receptor deduced from the two cDNAs and expression experiments, knowledge as to the functions of PAF and PAF receptor, and the pathogenic mechanisms of morbid states associated with PAF, is on the point of being obtained.

EP-A-0638648 discloses a monoclonal antibody specifically reacting with a PAF receptor. Hybridomas producing this monoclonal antibody are also disclosed, as are their deposits as FERM BP-4723 (Hybridoma PAF-R1④) and FERM BP-4722 (YM-PAFR) at the National Institute of Bioscience and Human Technology, Agency of Industrial Science and Technology, Japan. The generation process and characterisation of PAF-R1④ are described in detail in J. Biochem., 304:829-835 (1995). An antibody secreted by the hybridoma is designated an anti-(gpPAF-R¹⁵⁸⁻¹⁷³)AB-IV (Ab-IV) in this literature.

In this literature, it is described that the antibodies against the PAF receptor have specific avidity for both guinea pig and human PAF receptors. This may be very useful in development of medicines.

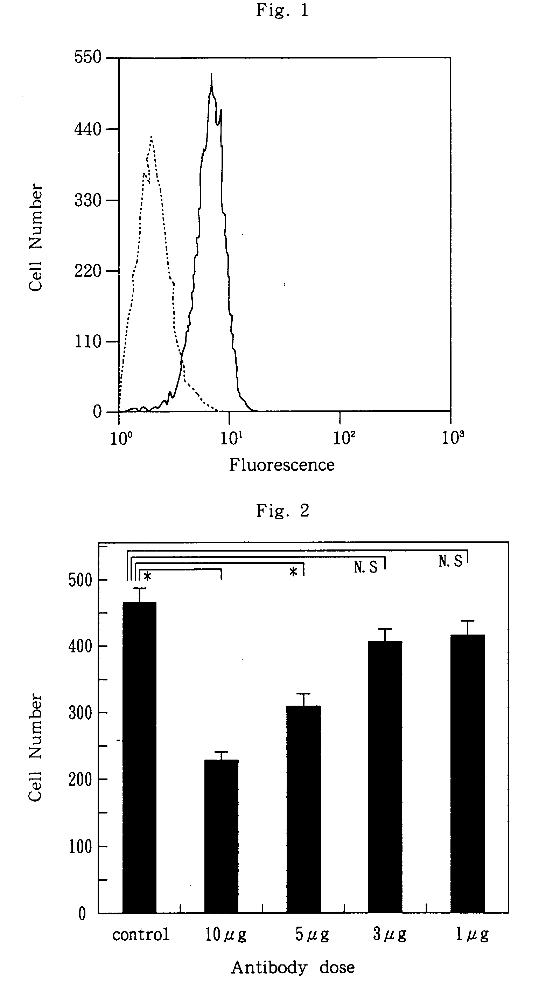

As described above, bronchial asthma which is one of allergic disease has proved for eosinophils to bear an important role from the results of its analysis. Therefore, the present inventors have considered that PAF has important effects on the chemotaxis and activity of eosinophils and thus analyzed eosinophils using antibodies against a PAF receptor. As a result, it has been found that eosinophils have a PAF receptor as illustrated in Fig. 1, and eosinophilic chemotaxis by PAF can be inhibited by an antibody against the PAF receptor as illustrated in Fig. 2. The present inventors have carried out an extensive investigation based on this finding. As a result, we have found a new effect that the antibody against the PAF can inhibit bronchial asthma, and that a composition comprising this antibody as an active ingredient is useful as an remedy (antiallergic agent) for allergic diseases such as bronchial asthma, thus leading to completion of the present invention.

According to the present invention, there is thus provided an antiallergic agent comprising, as an active ingredient, an antibody against a human PAF receptor or an active fragment thereof.

According to the present invention, there are also provided the following preferred embodiments.
1. The antiallergic agent which is suited for bronchial asthma.
2. The antiallergic agent wherein the antibody against the human PAF receptor is a monoclonal antibody.
3. The antiallergic agent wherein an epitope of the antibody against the human PAF receptor is situated in the extracellular third domain of the PAF receptor.
4. The antiallergic agent wherein the active fragment of the antibody against the human PAF receptor is at least one selected from the group consisting of F(ab')₂, Fab', Fab, Fv and recombinant Fv.
5. The antiallergic agent which can inhibit a delayed allergic reaction and an immediate allergic reaction.
6. The antiallergic agent which can inhibit infiltration of eosinophils and lymphocytes into bronchial tissue.
7. The antiallergic agent which can inhibit ECP release from eosinophils.

### BRIEF DESCRIPTION OF THE DRAWINGS

FIG. 1 is a graph illustrating that an antibody according to the present invention reacts with human eosinophils, and obtained by flow cytometry.

FIG. 2 is a graph illustrating an inhibitory effect on eosinophils chemotaxis induced by PAS brought about by administration of an anti-PAF receptor antibody.

FIG. 3 is a graph illustrating an inhibitory effect on ECP release from eosinophils induced by PAF brought about by administration of the anti-PAF receptor antibody.

FIG. 4 is a graph illustrating an effect to reduce the airway resistance of guinea pigs inhaled with PAF brought about by administration of the anti-PAF receptor antibody.

FIG. 5 is a graph illustrating an inhibitory effect on infiltration of eosinophils into the airway tissue of the guinea pigs inhaled with PAF brought about by administration of the anti-PAF receptor antibody.

FIG. 6 is a graph illustrating an inhibitory effect on infiltration of eosinophils into cell fractions in BALFs from the guinea pigs inhaled with PAF brought about by administration of the anti-PAF receptor antibody.

FIG. 7 is a graph illustrating an effect to reduce the airway resistance of model guinea pigs of asthma brought about by administration of the anti-PAF receptor antibody.

FIG. 8 is a graph illustrating an inhibitory effect on infiltration of eosinophils into the airway tissue of the model guinea pigs of asthma brought about by administration of the anti-PAF receptor antibody.

FIG. 9 is a graph illustrating an inhibitory effect on infiltration of eosinophils into the cell fractions in BALFs from the model guinea pigs of asthma brought about by administration of the anti-PAF receptor antibody.

### DETAILED DESCRIPTION OF THE INVENTION

An antibody against a PAF receptor (i.e., an anti-PAF receptor antibody) used as an active ingredient in the antiallergic agent according to the present invention is an antibody reacting with the PAF receptor which is a cell surface molecule. As the antibody against the PAF receptor, a monoclonal antibody specifically reacting with the PAF receptor is preferred. Such a monoclonal antibody can be obtained, for example, in accordance with the following process.
(1) A rodent is immunosensitized with a peptide of a PAF receptor;
(2) the spleen is taken out of the immune rodent to form a suspension of splenocytes;
(3) the suspension of the splenocytes is mixed with myeloma cells of a mouse in the presence of a hybridization accelerator to fuse both cells;
(4) the fused cells are diluted with a medium which does not grow unfused myeloma cells, to culture the fused cells, thereby sorting hybridomas produced by the fusion of the antibody-producing cells with the myeloma cells;
(5) the presence of an antibody in a supernatant in each of culture wells separately containing the hybridomas is confirmed using, as an indicator, the reactivity to the PAF receptor peptide;
(6) hybridomas, which produce the desired antibodies are selected, and the hybridomas are then cloned by the limiting dilution technique; and
(7) the desired antibodies are recovered from the culture supernatants of the monoclonal hybridoma cells.

Examples of the monoclonal antibody used as an active ingredient in the antiallergic agent according to the present invention include monoclonal antibodies produced by hybridomas which are being deposited as Deposit Nos. FERM BP-4723 (Hybridoma PAF-R1④ and FERM BP-4722 (YM-PAFR) in National Institute of Bioscience and Human-Technology, Agency of Industrial Science and Technology, Japan. In the monoclonal antibodies specifically reacting with the PAF receptor, epitopes of the antibodies against a human PAF receptor exist in the extracellular third domain of the PAF receptor.

A polyclonal antibody against the PAF receptor can be obtained, for example, in accordance with a process in which a rodent is immunosensitized with a peptide of a PAF receptor, and blood is collected from the immunosensitized rodent to separate a serum component, thereby obtaining the intended antiserum (an unpurified polyclonal antibody). The antiserum is preferably purified by a method known per se in the art to provide it as a purified polyclonal antibody.

The antibody against the PAF receptor is an immunoglobulin. An monoclonal antibody specifically reacting with the PAF receptor is a homogeneous immunogloblin. For example, there is an antibody which belongs to a class of IgM and the L chain of which is a κ chain, or an antibody which belongs to a subclass of IgG2a and the L chain of which is a *k* chain. The term "active fragment of an antibody against the PAF receptor" means a fragment of an immunoglobulin having antigen-antibody reaction activity which the anti-PAF receptor antibody has. Specific examples of such a fragment include F(ab')₂, Fab', Fab, Fv and recombinant Fv. These active fragments may be prepared from the immunoglobulin (anti-PAF receptor antibody) in accordance with a method known per se in the art. For example, the F(ab')₂ fragment can be obtained by digesting an immunoglobulin IgG with pepsin. The Fab' fragment can be obtained by reducing the F(ab')₂ fragment with a reagent such as 2-mercaptoethanol and alkylating the reduced product with monoiodoacetic acid. The Fab fragment can be obtained by the papain digestion of IgG. The Fv fragment can be obtained by bonding a variable region of H chain (V_{H}) to a variable region of L chain (V_{L}) by a noncovalent bond. The recombinant Fv fragment can be obtained by sequencing DNA as to genes corresponding to variable regions of H and L chains of an antibody from, for example, a hybridoma which produces a monoclonal antibody, thereby determining base sequences which code the variable region of H chain (V_{H}) and the variable region of L chain (V_{L}), respectively, and then integrating these DNA fragments in a vector to cause Escherichia coli or cells such as animal cells to produce a monovalent active antibody fragment having a structure of V_{H}-Linker-V_{L}.

The anti-PAF receptor antibody has a possibility that it may react with all the human, mouse, rat and guinea pig PAF receptors. For example, the monoclonal antibody obtained in the above-described process reacts with (recognizes) human eosinophils on which a human PAF has been expressed. The monoclonal antibody also reacts with cell-cultured mast cells on which a mouse or rat PAF receptor has been expressed. Accordingly, it is possible to infer a pharmacological effect on the human from the result of an animal experiment.

Guinea pigs used in Example of the present invention are very good models for analyses of asthma, allergy and inflammation. These guinea pigs show symptoms of asthma by inhaling ovalbumin (OVA) to sensitize them as described in Allergy, 37(10), pp. 980-991 (1988). More specifically, when the guinea pigs are sensitized by inhaling 10 mg/ml of OVA into them for 10 minutes per day over 10 days to sensitize them, and then inhaling the same amount of OVA twice every other week to sensitize them, they become good models for asthma. The anti-PAF receptor antibody is administered to these guinea pigs before the final sensitization with OVA to confirm its effect, thereby leading to completion of the antiallergic agent according to the present invention.

The antiallergic agent according to the present invention can inhibit a delayed allergic reaction (late bronchial asthma) and an immediate allergic reaction (immediate bronchial asthma). The antiallergic agent according to the present invention can also inhibit the infiltration of eosinophils and lymphocytes into bronchial tissue. Further, the antiallergic agent according to the present invention can inhibit ECP release from eosinophils.

In order to more enhance activity of the anti-PAF receptor antibody as an antiallergic agent, it is effective to convert the anti-PAF receptor antibody into a human or chimeric type. These techniques have been already described in known literature or patent publications and can be easily performed if hybridomas producing an antibody are in existence. Examples of the patent publications include EP-0120694, EP-0125023, EP-0171496, EP-A-0173494 and WO86101533, while examples of the literature include Nature, 322, pp. 323-327 (1988), Nature, 321, pp. 522-525 (1986) and Nature, 328, pp. 731-734 (1987). On the basis of these known techniques, the anti-PAF receptor antibody can be easily converted into the human or chimeric type.

Examples of the preparation form of the antiallergic agent according to the present invention include injections, tablets, capsules, suppositories, nebulae, cream compositions, poultices and ophthalmic solutions like the conventional drugs and medicinal compositions each comprising an active ingredient. In the case of, for example, an injection, a solution containing the antibody according to the present invention is prepared under sterile conditions. A stabilizer such as mannitol, an excipient and the like may be added as auxiliary ingredients as needed. This solution may be charged into ampules, vials and the like and used as it is. Alternatively, the solution may be lyophilized as needed. In the case where the antiallergic agent according to the present invention is a dry product, it may be dissolved in distilled water for injection or the like before its administration. With respect to an administration method, it is only necessary to select a suitable method from oral administration, intravenous injection, inhalation, percutaneous administration, instillation, topical administration, subcutaneous administration or the like for administration.

The antiallergic agent according to the present invention comprises, as an active ingredient, the antibody against a PAF receptor, or the active fragment thereof in a proportion of generally 0.1-100 wt.%, preferably 0.5-70 wt.%. A dose of the antiallergic agent according to the present invention is within a range of generally 0.001-1,000 mg, preferably 0.001-600 mg in terms of the active ingredient per day for an adult. It goes without saying that the above dose is only a standard by and large, and a dose may be suitably selected according to the age, sex and weight of a patient to be dosed, and moreover the kind and condition of a disease.

### ADVANTAGES OF THE INVENTION

The antiallergic agent according to the present invention is useful for treatment for inflammatory diseases including bronchial asthma. It is particularly useful for treatment for diseases in which eosinophils participate. The reason for it is that the antiallergic agent according to the present invention inhibits the infiltration of eosinophils and ECP release from the eosinophils. The diseases involved by eosinophils are classified as eosinophilia, and include allergic diseases including asthma, vermination, leukemia and fasciitis. In order to make the antiallergic agent according to the present invention more effective, it is effective to convert the anti-PAF receptor antibody into a humanized or chimeric type.

The merit of the anti-PAF receptor antibody as the antiallergic agent according to the present invention is that it can react with (recognize) all the human, mouse, rat and guinea pig PAF receptors. Namely, the results of experiments, which will be described subsequently, are effects which may be applied to the human as they are, and are very useful. With respect to side effects and the like of this remedy, it is predictable that the remedy is safe for the human if it is safe for guinea pigs, because the remedy reacts with any of the above kinds of animals.

### EMBODIMENTS OF THE INVENTION

The present invention will hereinafter be described in more detail by reference to the following Example. Example 1:

### (1) Staining of PAF receptor on eosinophil:

Following the same process as that described in Japanese Patent Application Laid-Open No. 101998/1995, staining was conducted using the same antibody as that described therein. More specifically, whether a monoclonal antibody produced by the hybridoma YM-PAFR deposited as FERM BP-4722 reacts with human eosinophils or not was determined.

Human eosinophils were separated in accordance with the following process.
1) peripheral Venous blood was collected into a disposable syringe containing heparin.
2) The peripheral venous blood was transferred to a 50-ml test tube to which a 6% dextran solution was added at a ratio of the dextran solution to the blood of 1:5 by volume to mix them by overturning. Incidentally, the 6% dextran solution was prepared from 0.150 M NaCl and 6% (w/v) Dextran T70 (product of Pharmacia) and sterilized by filtration to store it.
3) The resultant mixture was transferred to another 50-ml test tube gently in such a manner that bubbles were not formed, and the test tube was left at rest at 37°C for 60 minutes.
4) A supernatant containing leukocytes was transferred to a further 50-ml test tube to which a Pipes/FCS buffer was added. The resultant mixture was centrifuged for 10 minutes under conditions of 250 x g. The leukocytes were washed further twice with the Pepes/FCS buffer.

| | |
|---|---|
| Pipes/FCS buffer (pH 7.4): | |
| 0.110 M | NaCl |
| 5.0 mM | KCl |
| 40 mM | NaOH |
| 25 mM | piperazine-N,N'-bis-(2-ethane-sulfonic acid) |
| 5.4 mM | D-glucose |
| 1% (v/v) | heat-inactivated fetal calf serum (FCS). |

The buffer was sterilized by filtration and stored. Right before its use, deoxyribonuclease (product of Sigma) was added in a proportion of 30 mg/liter so as not to cause cellular aggregation.
(5) A Heavy solution and a Light solution were mixed in proper amounts, thereby preparing Percoll solutions whose specific gravities were 1.100, 1.090, 1.085 and 1.080, respectively. The mixing amounts were as follows.

| Specific gravity | Heavy Solution (ml) | Light solution (ml) |
|---|---|---|
| 1.100 | 8.43 | 1.57 |
| 1.090 | 7.45 | 2.55 |
| 1.085 | 6.96 | 3.04 |
| 1.080 | 5.78 | 4.22 |
| 1.070 | 5.49 | 4.51 |

Incidentally, the Heavy solution and Light solution have the following compositions, respectively.

| | |
|---|---|
| Heavy solution of Percoll: | |
| (pH 7.35-7.45) | |
| 500 ml | Percoll stock solution (product of Pharmacia) |
| 46.5 ml | Pipes buffer at a concentration ten times of the amount of the stock solution |
| 12 ml | purified distilled water. |

The pH of the Heavy solution is adjusted with concentrated hydrochloric acid.

| | |
|---|---|
| Osmotic pressure | 280-290 mOsm/kg |
| Specific gravity | 1.122-1.123 |

| | |
|---|---|
| Light solution of Percoll: | |
| (pH 7.35-7.45) | |
| 50 ml | Heavy solution |
| 450 ml | Pipes buffer (containing no FCS, the pH was not adjusted). |

The pH of the Light solution is adjusted with 10N NaOH.

| | |
|---|---|
| Osmotic pressure | 280-290 mOsm/kg |
| Specific gravity | 1.020-1.021 |

Both solutions were sterilized by filtration.
6) The Percoll solutions whose specific gravities were 1.100, 1.090, 1.085 and 1.080 were stacked in amounts of 1.5, 3.0, 3.0 and 3.0 ml, respectively, in a 16-ml polycarbonate test tube. The stacking was conducted gently using a peristaltic pump.
7) The washed leukocytes were suspended in a Percoll solution whose specific gravity was 1.070 so as to give a cell concentration of 5 x 10⁷ cells/ml. Two milliliters of the suspension were stacked on the specific gravity-gradient prepared in the step 6).
8) Centrifugation was conducted for 20 minutes under conditions of 10°C and 1600 x g. The centrifugation was performed by using a fixed angle rotor so as not to shock the specific gravity-gradient and actuating a slowly accelerating device to brake off.
9) After completion of the centrifugation, a needle having a right-angled cut edge was gently inserted to the bottom of the test tube to gently recover the solutions by a roller pump connected to the needle. At this time, when a fractionator is connected to the pump, fractions can be automatically recovered in 1-ml portions. Since the whole volume was 12.5 ml, 13 fractions were obtained by the fraction recovery in 1-ml portions.
10) After the number of eosinophils in each of the respective fractions was counted, fractions high in purity were collected in a test tube and washed twice with the Pipes/FCS buffer to use experiments. Since the third to sixth fractions are generally high in purity, such fractions were recovered in this time.

After collecting the eosinophils thus prepared, the cells were put in portions of 1 x 10⁶ cells into Fischer tubes, to which 50 µg/ml of the antibody purified was added in an amount of 100 µl to react them for 20 minutes on an ice bath. Each of the reaction mixtures was centrifugally washed with PBS (3000 rpm, 1 minute, 3 times) and then added with 100 µl of FITC-anti-mouse Ig's (product of Caltag Company, diluted to 1:100) to react them for 20 minutes on an ice bath. The reaction mixture was centrifugally washed twice with PBS to suspend it in 200 µl of PBS, thereby conducting measurement by FACScan.

As a result, as illustrated in FIG. 1, it was revealed that the monoclonal antibody produced by the hybridoma YM-PAFR reacts with PAF-R on the human eosinophils. More specifically, FIG. 1 is an FACS graph illustrating the reaction of the human eosinophils with the anti-PAF receptor antibody. In the drawing, a solid line indicates the case where the anti-PAF receptor antibody was added, while a dotted line indicates the case where no antibody was added.

### (2) Inhibitory effect on eosinophilic migration induced by PAF brought about by administration of anti-PAF receptor antibody :

Upper parts of chemotaxis chambers were charged with 1 x 10⁶ cells/ml of the peripheral eosinophils prepared as described above in an amount of 200 µl. To these upper parts, the anti-PAF receptor antibody was added to give concentrations of 10 µg/ml, 5 µg/ml, 3 µg/ml and 1 µg/ml respectively. For the sake of comparison, an upper part containing no antibody was provided as a control. Each of the upper parts of the chambers was separated from the lower part by a filter. In the lower part, a culture solution containing PAF was contained. PAF was prepared by dissolving it in ethanol, and then diluting the ethanol solution with physiological saline containing 2.5% BSA to give a final concentration of 3 x 10⁻⁸ M. This dilute solution was added in an amount of 200 µl/chamber.

Thereafter, culture was conducted at 37°C for 3 hours, and cells present in an upper portion of the filter were removed by washing with PBS using a pipette. The filter was taken out. Thereafter, cells present in a lower portion of the filter were fixed with a solution composed of ethanol and saturated corrosive sublimate in equal amounts. At this time, the fixation was conducted with the surface of the filter on the side of the lower part turned up. The fixation was performed for 12 hours. Thereafter, staining was performed to count the number of the stained cells present in the lower portion of the filter through a microscope. The staining was conducted in accordance with the following process.

### Staining process:

(1) After fixed for 12 hours, the filter was stained with the following pints in mind.
   1. The filter is taken out of the fixative to wash it with water for 10 minutes.
   2. The filter is stained with hematoxylin for 10 minutes.
   3. The thus-stained filter is washed with water for 2 minutes.
   4. The filter is immersed for 5 seconds in 70% ethanol containing 1% HCl.
   5. The filter is washed with water for 10 minutes.
   6. The filter is stained with chromotrope for 30 seconds.
   7. The thus-stained filter is washed with water for 2 minutes.
   8. The filter is dehydrated with 100% ethanol (3 times each for several seconds).
   9. The filter is immersed for 2 minutes in n-propyl alcohol.
   10. The filter is immersed for 2 minutes in a solution composed of n-propyl alcohol and xylol in equal amounts.
   11. The filter is cleared with xylene for at least 5 minutes.
(2) The stained filter was occluded with Canada balsam. At this time, the filter was placed on a slide glass with the surface of the filter on the side of the lower part turned up to occlude.
(3) The number of migrated cells was counted in the predetermined field number (10-20 visual fields) through a microscope (400 magnifications). Since a nitrocellulose filter has a thickness of 130 µm and is hence greater in cellular migration distance upon chemotaxis compared with a polycarbonate filter (thickness: 15 µm), it is easy to distinguish chemotaxis from chemokinesis and also high in reproducibility. Therefore, it is desirable to use this filter in a migration inhibition experiment. The number of cells migrated up to 8 µm in depth from the lower surface of the filter was counted to regard the total number as a migration activity.
(4) Each chamber after used was washed by means of, for example, a chemotaxis chamber cleaning apparatus.

The results are illustrated in FIG. 2.

FIG. 2 is a graph illustrating the inhibitory effect on eosinophilic migration induced by PAF brought about by administration of the anti-PAF receptor antibody. As shown in FIG. 2, it was revealed that the migration of eosinophils is inhibited in dependence on the concentration of the antibody (in FIG. 2, *: P < 0.05, n = 3, mean ± SE).

### (3) Inhibitory effect of anti-PAF receptor antibody on ECP release from eosinophils stimulated by PAF

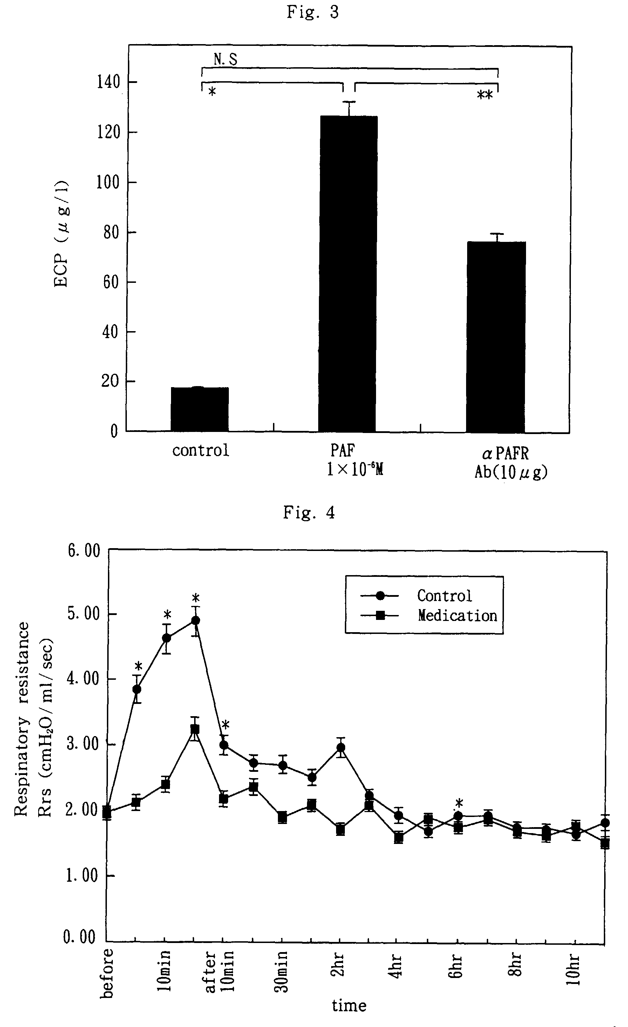

After the human peripheral eosinophils prepared as described in the experiment (1) were adjusted to give an amount of 1 x 10⁶ cells/ml in a 10% FCS·RPMI1604 medium, they were added in 1-ml portions to 9 wells of a 24-well microplate. Three wells of these wells were used as control to which no other substances were added. PAF was added in an amount of 1 x 10⁻⁶ M to other three wells. Finally, 1 x 10⁻⁶ M of PAF and 10 µg of the anti-PAF receptor antibody (αPAFRAb) were added to the remaining three wells. After 30 minutes, the amounts of ECP in culture supernatants were measured by means of an RIA kit (product of Pharmacia). As a result, as illustrated in FIG. 3, it was found that the release of ECP from eosinophil cells is significantly inhibited by the addition of the anti-PAF receptor antibody. FIG. 3 is a graph illustrating the inhibitory effect on ECP release from the eosinophils induced by PAF brought about by administration of the anti-PAF receptor antibody (in FIG. 3, *: P < 0.05, n = 3, mean ± SE).

### (4) Effect of anti-PAF receptor antibody to reduce airway resistance of guinea pigs stimulated by PAF:

The anti-PAF receptor antibody was intravenously administered in a proportion of 4 mg/kg to three Hartley guinea pigs in advance. Upon elapsed time of 1 hour after the administration of the antibody, 100 µg/ml of PAF were continuously inhaled into the three guinea pigs administered with the antibody and 3 guinea pigs administered with no antibody for 20 minutes at a flow rate of 6 liters/min.

The airway resistance was measured after 5 minutes, 10 minutes, 20 minutes from the beginning of the PAF inhalation, and further after 10 minutes, 20 minutes, 30 minutes, 1 hour, 2 hours, 3 hours, 4 hours, 5 hours, 6 hours, 7 hours, 8 hours, 9 hours and 10 hours from completion of the PAF inhalation. The results are summarized in FIG. 4. Namely, FIG. 4 is a graph illustrating the effect to reduce the airway resistance of the guinea pigs inhaled with PAF brought about by administration of the anti-PAF receptor antibody. As apparent from FIG. 4, it was revealed that the group administered with the anti-PAF receptor antibody is low in airway resistance compared with the group administered with no antibody, and asthmatoid fit on such a group is hence inhibited (in FIG. 4, *: P < 0.05, n = 3, mean ± SE).

### (5) Inhibitory effect of anti-PAF receptor antibody on infiltration of eosinophils into the airway tissue of guinea pigs stimulated by PAF:

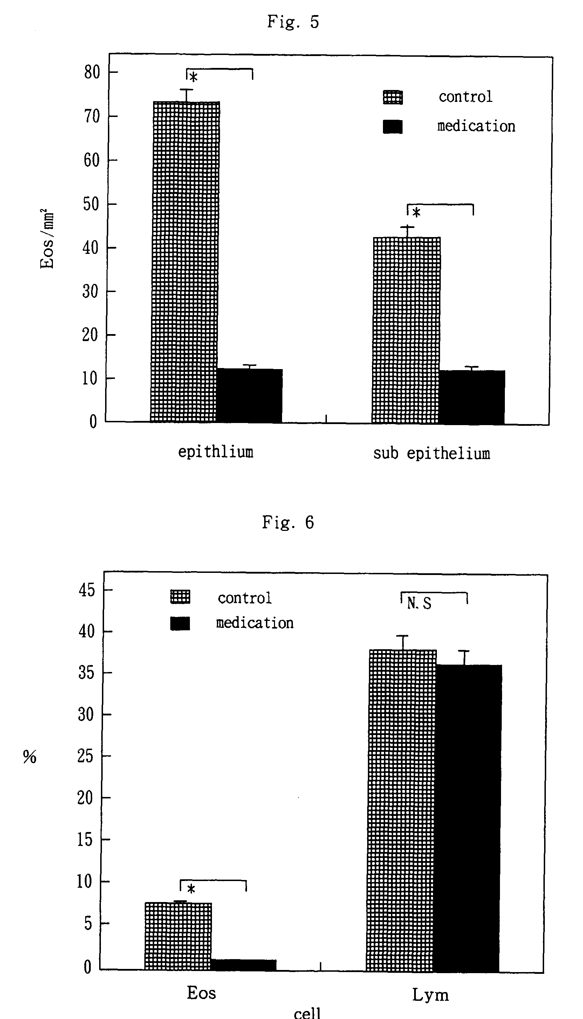

With respect to the guinea pigs used in the experiment (4), the number of eosinophils infiltrated into the airway tissue was counted after 24 hours from completion of the PAF inhalation (PAF stimulation). More specifically, after the airway tissue was fixed with formalin, it was embedded in paraffin to prepare segments. Staining was conducted on each three segments by Hansel, hematoxylin-eosin and Giemsa staining methods to count the number of eosinophils in accordance with the Hansel staining. The results are illustrated in FIG. 5. FIG. 5 is a graph illustrating the inhibitory effect on infiltration of eosinophils into the airway tissue of the guinea pigs inhaled with PAF brought about by administration of the anti-PAF receptor antibody. As apparent from FIG. 5, it was revealed that the infiltration of the eosinophils into both respiratory mucous epithelium and subcutaneous tissue is significantly inhibited (in FIG. 5, *: P < 0.05, n = 3, mean ± SE).

### (6) Inhibitory effect of anti-PAF receptor antibody on infiltration of eosinophils into cells in BALFs from guinea pigs stimulated by PAF:

With respect to the guinea pigs used in the experiment (4), the numbers of cells in BALFs were compared with each other after 24 hours from completion of the PAF stimulation. Each of BALFs was washed three times with 10 ml of physiological saline and then recovered for use. After the fluid was centrifuged, the cells in the fluid were stained in accordance with the Diff·Quick staining to count them. As a result, as illustrated in FIG. 6, it was revealed that the infiltration of the eosinophils into BALF is significantly inhibited by the administration of the antibody. FIG. 6 is a graph illustrating the influence on the cell fractions in BALFs from the guinea pigs stimulated by PAF by administration of the anti-PAF receptor antibody (in FIG. 6, *: P < 0.1, n = 3, mean ± SE).

### (7) Effect of anti-PAF receptor antibody to reduce airway resistance of experimental asthmatic guinea pigs sensitized with ovalbumin:

Ovalbumin was continuously inhaled for 10 minutes in an amount of 10 mg/ml in six Hartley guinea pigs in advance to sensitize them. After the sensitization was first conducted for 10 minutes once a day over 10 days, additional sensitization was performed for 10 minutes after a week and after two weeks to provide model guinea pigs. Those showing a titer of at least 320 viewing from PCA reaction were used as the model guinea pigs.

The anti-PAF receptor antibody was intravenously administered in a proportion of 4 mg/kg to half three asthmatic guinea pigs thereof. Upon elapsed time of 1 hour after the administration of the antibody, 10 mg/ml of ovalbumin were continuously inhaled for 10 minutes into the three guinea pigs.

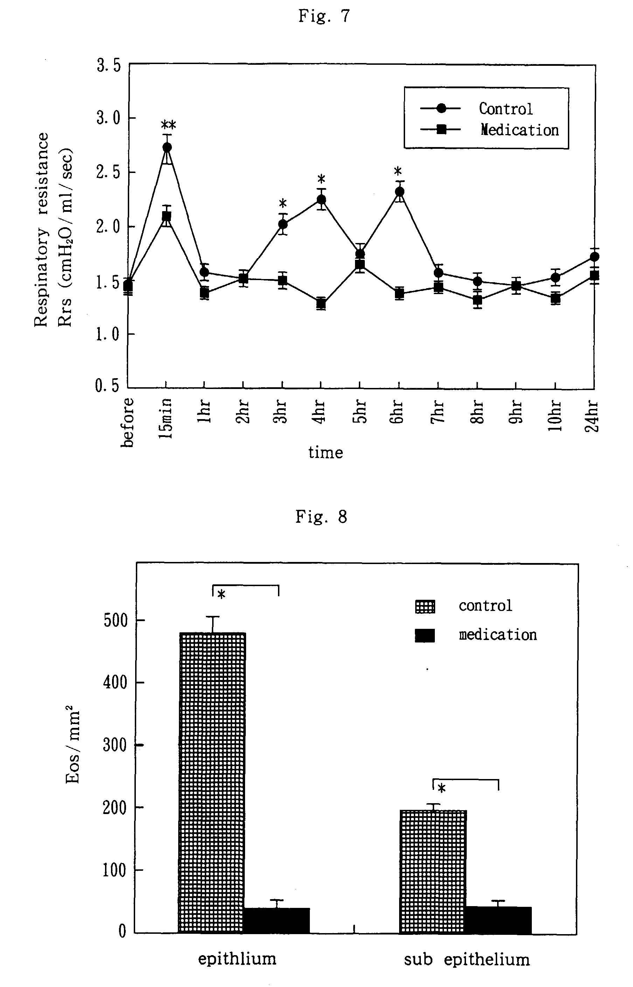

The airway resistance was measured after 15 minutes, 1 hour, 2 hours, 3 hours, 4 hours, 5 hours, 6 hours, 7 hours, 8 hours, 9 hours and 10 hours from beginning of the inhalation. The results are summarized in FIG. 7. Namely, FIG. 7 is a graph illustrating the effect to reduce the airway resistance of the model guinea pigs of asthma brought about by administration of the anti-PAF receptor antibody. As apparent from FIG. 7, it was revealed that the airway resistance of the group administered with the anti-PAF receptor antibody is reduced compared with the group administered with no antibody. This phenomenon significantly inhibited both immediate asthmatic response, which appeared as a peak after 15 minutes, and late asthmatic response, which appeared as a peak after 4-6 hours (in FIG. 7, *: P < 0.05, **: P < 0.1, n = 3, mean ± SE).

### (8) Inhibitory effect of anti-PAF receptor antibody on infiltration of eosinophils into the airway tissue of experimental asthmatic guinea pigs sensitized with ovalbumin:

With respect to the guinea pigs used in the experiment (7), the number of eosinophils infiltrated into the airway tissue was counted after 24 hours from completion of the ovalbumin inhalation. The tissue was prepared for use by fixing with formalin, and then embedding it in paraffin to prepare each three segments in the same manner as in the experiment (5). Staining was conducted on each three segments by Hansel, hematoxylin-eosin and Giemsa staining methods to count the number of eosinophils in the same manner as in the experiment (5). As a result, as apparent from FIG. 8, it was revealed that the infiltration of the eosinophils into both respiratory mucous epithelium and subcutaneous tissue is significantly inhibited. FIG. 8 is a graph illustrating the inhibitory effect on infiltration of eosinophils into the airway tissue of the model guinea pigs of asthma brought about by administration of the anti-PAF receptor antibody (in FIG. 8, ∗: P < 0.05, n = 3, mean ± SE).

### (9) Inhibitory effect of anti-PAF receptor antibody on infiltration of eosinophils into cells in BALFs from experimental asthmatic guinea pigs sensitized with ovalbumin:

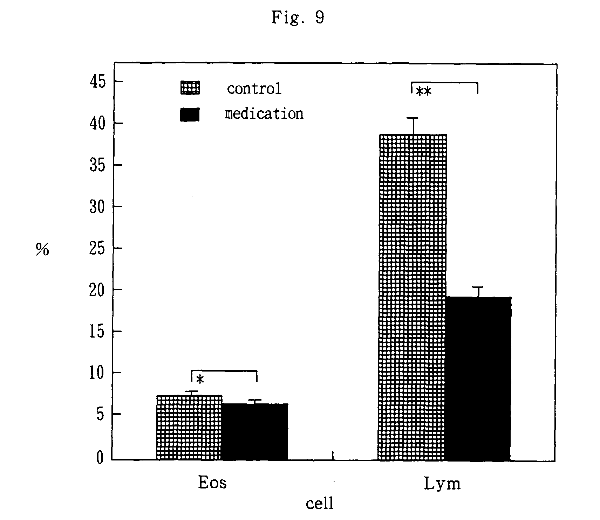

With respect to the guinea pigs used in the experiment (7), the numbers of cells in BALFs were compared with each other after 24 hours from completion of the ovalbumin inhalation. Each of BALFs was washed with physiological saline, and the cells were stained in accordance with the Diff·Quick staining in the same manner as in the experiment (6). As a result, as illustrated in FIG. 9, it was revealed that the infiltration of not only the eosinophils but also lymphocytes into BALF is significantly inhibited by the administration of the anti-PAF receptor antibody. FIG. 9 is a graph illustrating the influence on the cell fractions in BALFs from the model guinea pigs of asthma by administration of the anti-PAF receptor antibody (in FIG. 9, *: P < 0.5, **: P < 0.3, n = 3, mean ± SE).

## Claims

1. Use of an antibody against a human PAF receptor or an active fragment thereof, for the manufacture of a medicament for use as an anti-allergic agent.

2. Use according to claim 1, wherein the antibody is a monoclonal antibody.

3. Use according to claim 2 wherein the monoclonal antibody is obtainable from the hybridoma deposited as Deposit No. FERM BP-4723 (Hybridoma PAF R1④) or FERM BP-4722 (YM-PAFR) in National Institute of Bioscience and Human Technology, Agency of Industrial Science and Technology, Japan.

4. Use according to any preceding claim, wherein an epitope of the antibody is located in the extracellular third domain of the PAF receptor.

5. Use according to any preceding claim, wherein the active fragment of the antibody is selected from F(ab')₂, Fab', Fab, Fv and recombinant Fv.

6. Use according to any preceding claim, wherein the antibody can inhibit delayed allergic reaction and immediate allergic reaction.

7. Use according to any preceding claim, wherein the antibody can inhibit infiltration of eosinophils and lymphocytes into bronchial tissue.

8. Use according to any preceding claim, wherein the antibody can inhibit ECP release from eosinophils.

9. Use according to any preceding claim, for the treatment of bronchial asthma.
